# EUROPEAN PATENT SPECIFICATION

(11) **EP 0 969 004 B1**
(45) Date of publication and mention of the grant of the patent: **10.12.2003**
(21) Application number: 99110534.7
(22) Date of filing: 01.06.1999
(51) Int. Cl.: C07D 251/70, A61K 7/42, A61K 7/48, C08K 5/3492

(54) **2,4,6-Trianilino-1,3,5-triazine derivatives, their preparation and their use as sunscreens against UV-B sun radiations**
2,4,6-Trianilino-1,3,5-triazin Derivate, deren Herstellung und deren Verwendung als Sonnenschutzmittel gegen UV-B Strahlen
Dérivés de 2,4,6-trianilino-1,3,5-triazine, leur préparation et leur utilisation comme filtres solaires contre les rayonnements UV-B

(30) Priority: 05.06.1998 IT MI981266
(43) Date of publication of application: 05.01.2000
(73) Proprietor: 3V SIGMA S.p.A, 20121 Milano (IT)
(72) Inventor: Raspanti, Giuseppe, 24121 Bergamo (IT); Zanchi, Giorgio, 24121 Bergamo (IT)
(74) Representative: Minoja, Fabrizio, Dr.

(56) References cited:
- EP-A- 0 046 139
- EP-A- 0 087 098
- EP-A- 0 517 104
- EP-A- 0 570 838
- EP-A- 0 796 851
- EP-A- 0 818 450
- US-A- 2 328 961

## Description

The present invention relates to novel 1,3,5-trianilinotriazine derivatives and to the use thereof as protecting agents from UV-B sun radiations and as agents useful in the photostabilization of synthetic polymers.

1,3,5-Trianilino triazine derivatives as UV-A/B filters and as photostabilising agents are disclosed in EP 0 756 851 and in EP 0 818 450.

More precisely, the present invention relates to compounds of general formula I: 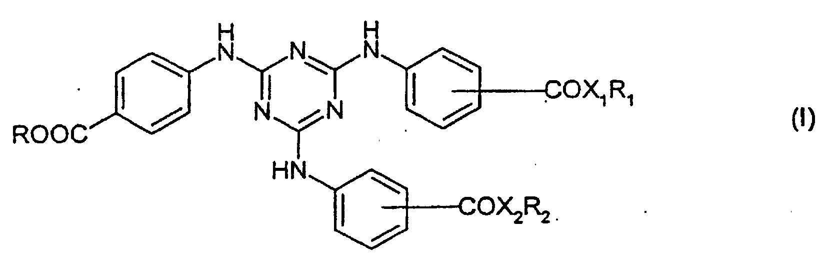 wherein R, R₁ and R₂ are independently straight or branched (C₁₋₁₈)alkyl; (C₅₋₁₂)cycloalkyl, optionally substituted with 1 to 3 straight or branched (C₁₋₄) alkyl groups;
X₁ and X₂ are independently oxygen or the group NH;
with the proviso that the two CO-X₁-R₁ and CO-X₂-R₂ groups cannot simultaneously be at the para position.

Examples of (C₁₋₄)alkyl, (C₁₋₈)alkyl and (C₁-₁₈) alkyl groups comprise methyl, ethyl, isopropyl, tert.butyl, 3,3-dimethylpropyl, n-hexyl, 2-ethylbutyl, 2-ethylhexyl, 1,1,3,3 -tetramethylbutyl, n-decyl, n-pentadecyl and n-hexadecyl.

(C₅₋₈)Cycloalkyl and (C₅₋₁₂)-cycloalkyl herein essentially mean cyclopentyl, cyclohexyl, cycloheptyl, cyclodecyl and analogues.

A preferred group of compounds of the invention comprises compounds of formula I in which R, R₁ and R₂ are independently straight or branched (C₁₋₁₈)alkyl, or (C₅₋₁₂)-cycloalkyl, optionally substituted with 1 to 2 straight or branched (C₁₋₄)-alkyl groups, X₁ and X₂ are oxygen or NH, one of the two CO-X₁-R₁ or CO-X₂-R₂ groups is at the para position and the other is at a different position from the para position.

A second preferred group of compounds of the invention comprises compounds of formula I in which R and R₁ are simultaneously straight or branched (C₁₋₁₈)-alkyl, or (C₅₋₁₂)-cycloalkyl, optionally substituted with 1 to 2 straight or branched (C₁₋₄)alkyl groups, R₂ is straight or branched (C₁₋₁₈)alkyl, or (C₅₋₁₂)cycloalkyl, optionally substituted with 1 to 2 straight or branched (C₁₋₄)alkyl groups, X₁ is oxygen, X₂ can be oxygen or NH, the group CO-X₁-R₁ is at the para position and the group CO-X₂-R₂ is at a different position from the para position.

It is well known that sunlight ultraviolet radiation exerts a noxious action on human skin, and it causes degradation of synthetic polymers as well. By use of the so-called sunscreens, capable of adsorbing sunlight UV radiation, the harmful effects such as aging of the skin or, in case of synthetic polymers, the degradation thereof, can be prevented or at least delayed.

The radiations ranging from 290 to 320 nm, i.e. UV-B radiations, are particularly noxious to human skin, in that they are responsible for dangerous erythemas. A number of compounds have been investigated and tested for any effects against such UV action, particularly p-hydroxycinnamic and p-dimethylaminobenzoic acids esters, benzotriazoles and hydroxybenzophenones. The absorbance of UV-B radiation of these substances is, however, rather poor. Better results were obtained with particular triazine sunscreens (EP A2 0 087 098, EP A1 0 570 838 and EP B1 0 517 104). Nevertheless, the problems related to photostability of the sunscreen itself and its solubility in the oily solvents used, for example, in cosmetics, are yet to be solved.

It has now surprisingly been found that the compounds according to the present invention absorb very intensely the UV-B radiation, and show very good solubility in the oily solvents used in cosmetics. Furthermore, the compounds of the invention have also proved to effectively protect cosmetic formulations and synthetic polymers from sunlight-induced degradation, as well as to have poor toxicity.

Therefore, a further object of the present invention is the use of compounds of formula I as sunscreens for the preparation of cosmetic compositions for protecting the skin from sunlight, and as photostabilizers for synthetic polymers.

A further object of the invention is the use of the compounds of the invention as sunscreens for the cosmetic treatment of the skin.

A still further object of the invention are cosmetic compositions containing one or more compounds of formula I.

Finally, a further object of the invention are synthetic polymers compositions containing one or more compounds of formula I.

Compounds of formula I are prepared by conventional procedures, known to those skilled in the art. For example, a 1,3,5-trihalotriazine, preferably 1,3,5-trichlorotriazine, can be reacted with a compound of formula III 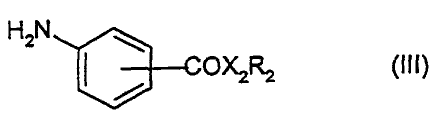 wherein X₂ and R₂ are defined as above, in reaction conditions such that only one halogen atom of 1,3,5-trichlorotriazine is replaced. A compound of formula IV 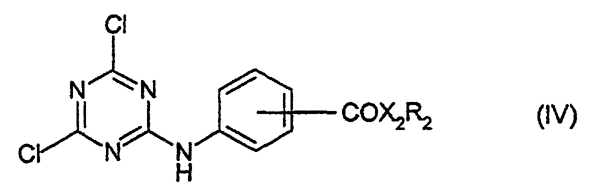 is obtained, wherein X₂ and R₂ have the above defined meanings, in which the two chlorine atoms can be replaced first with a compound of formula V or VI and, subsequently, with a compound of formula VI or V 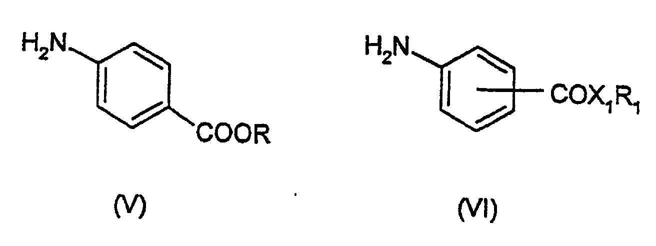 wherein R, R₁ and X₁ are as defined above.

Compounds of formulae III, V and VI are known in literature, or they can be prepared according to methods described in literature, starting from the corresponding aminobenzoic acids.

The reaction between trihalotriazine and aminobenzoic acids to replace only one halogen atom is also described in literature (for example, in EP 0 570 838 A1). This is carried out at temperatures ranging from about -5°C to about 10°C, in the presence of water, organic solvents such as aliphatic ketones, aromatic hydrocarbons, dioxane or mixtures thereof, with or without water. The resulting compound of formula IV can be recovered and characterized, if desired, or it can be directly reacted with a compound of formula V or VI, to replace a further chlorine atom. The resulting compound can also be recovered and characterized, or be directly reacted with a compound of formula VI or V, to obtain the desired final compounds of formula I.

According to one aspect of the present invention, when compounds of formula I are desired in which the CO-X₂-R₂ group is at ortho or meta position, X₁ is oxygen, R and R₁ have both the same meaning and the CO-X₁-R₁ group is at the para position, a compound of formula IV is reacted with about two to about four molar equivalents of a compound of formula V, or of a compound of formula VI in which X₁ is oxygen and CO-X₁-R₁ group is at the para position, in the presence of an organic solvent such as acetonitrile, aliphatic ketones, ethers of C₄-C₁₀ aliphatic hydrocarbons, dioxane, C₇-C₁₂ aliphatic hydrocarbons, aromatic hydrocarbons, or mixtures thereof. The reaction is usually carried out at the boiling temperature of the reaction mixture, for a time ranging from about two to about twelve hours, anyway depending on the solvent system used.

In any cases, the reaction of trihalotriazines and, more particularly, that of trichlorotriazine, for replacing the three halogen atoms with amino residues, which can be the same or different, is known in literature (Chem. Abs. 61, 3233d, 1964; Chem. Abs. 65, 15545g, 1966; Chem. Abs. 78, 112679, 1973; Chem. Abs. 81, 93087, 1974).

The final compounds are recovered and purified according to conventional procedures.

As stated above, the compounds of the invention are useful as UV-B sunscreens. The suitably formulated compounds of the invention exert their skin-protecting activity once applied on the skin area exposed to radiations in a suitable amount. This amount will be determined by those skilled in the art, depending on the specific extinction coefficient E₁¹ of the selected compound of formula I. Said coefficient is an index of the protection efficacy.

The cosmetic compositions which are one of the objects of the invention contain an effective amount of at least one compound of formula I, in combination with conventional carriers and excipients. By way of example, said compositions can be solutions, lotions, emulsions of the water-in-oil, oil-in-water, water-oil-water or oil-water-oil type; or they can also be in the form of gels, lipsticks, aerosol, etc. They are prepared by formulating the ingredients usually employed, such as oils, fats, emulsifiers, moisturizing agents, humectants, emollients, preservatives, surfactants, thickening agents, anti-foaming agents, perfumes, pigments, dyes and other else such as alcohols, polyols, electrolytes, silicone derivatives. Representative examples, of oily solvents in which the compounds of the invention show high solubility and which can be used in the cosmetic compositions are paraffin, mineral oils, oils, butters and natural waxes, silicone oils, esters of mono- or di-carboxylic, saturated or unsaturated, straight or branched aliphatic acids, or of aromatic or alkylaromatic acids containing from 1 to 25 carbon atoms with C₁-C₂₅ mono or polyhydroxylated aliphatic alcohols, C₆-C₃₅ alcohols, ethers of fatty alcohols from 8 to 40 carbon atoms, glycol butylethers, butylether esters, N,N-diethylmethylbenzamides, ethyl 1-(N-acetyl-N-butyl)-propionate, and mixtures thereof.

It has surprisingly been found that is possible to obtain solutions comprising from about 10 to about 45% by weight of one or more compounds of the invention and from about 90 to about 55% by weight of one or more oily solvents mentioned above.

The present invention also comprises the protection of the cosmetic compositions themselves from UV radiation by use of the compounds of formula I. In this case, it is a matter of compositions whose components can undergo degradation or undesired colouring induced by light, such as shampoos and hair lacquers, hair dressing lotions, hair-dyeing compositions, make-up formulations, as nail lacquers, foundation, lipsticks.

The cosmetic compositions according to the present invention can comprise one or more compounds of formula I in amounts ranging from about 0.1 to about 20%, preferably from about 0.5 to about 15%, by weight on the total weight of the formulation. They can comprise, in addition to the compounds of formula I, other complementary sunscreens active against sun radiations. Representative examples, of these sunscreens are 3-(4-methylbenzylidene)-camphor; 2-ethylhexyl-(4-dimethylamino)-benzoate; 2-ethylhexyl-4-methoxycinnamate; isoamyl-4-methoxycinnamate; menthyl salicylate; octyl salicylate; 2-ethylhexyl-2-cyano-3,3-diphenylacrylate; 2,4,6-tris-[(4-carbo-2-ethylhexyloxy)-anilino)]-1,3,5-triazine; 2,4-bis-[(4-carbo-2-ethylhexyloxy)anilino]-6-[(p.-tert.butylamino-carbonyl)amino]-1,3,5-triazine; 4-methoxy-4'-tert.butyl-dibenzoyl-methane; 2-hydroxy-4-methoxybenzophenone; 2-phenyl-benzimidazol-5-sulfonic acid and the salts thereof; 2-hydroxy-4-methoxybenzophenone-5-sulfonic acid and the salts thereof; terephthalydene-3,3'-dicamphor-10,10'-disulfonic acid and the salts thereof. Moreover, the cosmetic compositions of the present invention, in addition to one or more compounds of formula I, optionally together with one or more of the above mentioned sunscreens, can also contain inorganic sunscreens commonly used in cosmetics; such as finely micronized titanium dioxide, zinc oxide, silica or aluminium oxide, and vitamins, such as vitamins of the groups A, B, C, D, tocopherols such as vitamin E, and derivatives thereof.

Both compounds of the invention and the above mentioned cosmetic compositions have shown an SPF (Sun Protection Factor) not below 2. SPF is an index of the sun protecting power of a sunscreen or of a cosmetic formulation.

A further object of the present invention is the use of compounds of formula I as photostabilizers for synthetic polymers. Polymeric materials which can be protected from UV radiations are, for example, polyethylene, polypropylene, polystyrene, polybutadiene, polyisoprene and copolymers thereof, polyvinyl acetate and copolymers thereof, particularly with polyethylene, polyesters such as polyethylene terephthalate, polyamides such as Nylon 6 and Nylon 6,6, polyurethans, polyacrylates, polymethacrylates, polyvinyl chloride.

Compounds of formula I can be incorporated in the polymers to stabilize according to conventional procedures; for example, they can be mixed with the polymer in a suitable mixer, or be added in the form of solution or suspension in a suitable solvent, such as methanol, ethanol, acetone or chloroform, and then removing the solvent after mixing with the polymer in its various forms. Alternatively, compounds of formula I can be added to the polymer to be stabilized during the preparation of the polymer in its various forms, for example in the last preparation step.

For these purposes, compounds of formula I can also be used in combination with other stabilizers and additives conventionally used for polymers, such as phenolic antioxidants; phosphites; Ca, Mg, Zn, Al, Ba, Sn salts with C₈₋₂₀ organic acids, hindered amines, particularly those containing the 2,2,6,6-tetramethylpiperidine group in their structure, other types of UV-absorber, such as benzotriazoles or benzophenones, plasticizers, lubricants, flame-retardants, titanium dioxide.

As a rule, an amount ranging from about 0.01 to about 5% by weight on the weight of the polymeric material, preferably from about 0.05 to about 2%, is sufficient.

The following examples further illustrate the invention.

### A) - Preparation of compounds of formula IV

Example 1 - 2-Ethylhexyl-N-(4,6-dichloro-1,3,5-triazin-2-yl)-anthranylate - A mixture of 42.6 g of 1,3,5-trichlorotriazine and 22 g of sodium bicarbonate in 450 ml of acetone, cooled to 0°C, was slowly added with 64.7 g of 2-ethylhexyl anthranylate, keeping the temperature between 0 and 5°C by cooling with ice bath. After completion of the addition, the mixture was stirred for a further 30 minutes, added with 175 ml of water and stirred for one further hour, keeping temperature between 0 and 5°C. A precipitate formed, which was filtered, washed 4 times with water, subsequently with acetone and finally dried in oven. 84 g of the title product were obtained, having melting point 136-138°C.

Operating substantially as described in the above example, by reacting 1,3,5-trichlorotriazine with the suitable compound of formula II, the following compounds of formula III were prepared.

Example 2 - 3-[(4,6-Dichloro-1,3,5-triazin-2-yl)amino]-benzoic acid 2-ethylhexyl ester -
from 1,3,5-trichlorotriazine and 3-aminobenzoic acid 2-ethylhexyl ester, M.p.: 151-52°C.

Example 3 - 3-[(4,6-Dichloro-1,3,5-triazin-2-yl)amino]-benzoic acid tert.butylamide -
from 1,3,5-trichlorotriazine and 3-aminobenzoic acid tert.butylamide. M.p.: 206.5-08 °C.

Example 4 - 2,2-Dimethylpropyl-N-(4,6-dichloro-1,3,5-triazin-2-yl)-anthranylate -
from 1,3,5-trichlorotriazine and 2,2-dimethylpropyl anthranylate. M.p.: 195-96.5 °C.

### B) - Preparation of compounds of formula I

Example 5 - A solution of 11.9 g of the compound of Example 1 in 150 ml of xylene was added with 15.7 g of 2-ethylhexyl 4-aminobenzoate. The reaction mixture was stirred and refluxed for 3 hours. The formed hydrochloric acid was neutralized with diluted aqueous sodium hydroxide. After evaporating to dryness the xylene solution, a residue was obtained which was crystallized from n-hexane, to give 16 grams of a compound of the following formula, having melting point 77-80°C and E₁¹ 1024 at 311 nm.

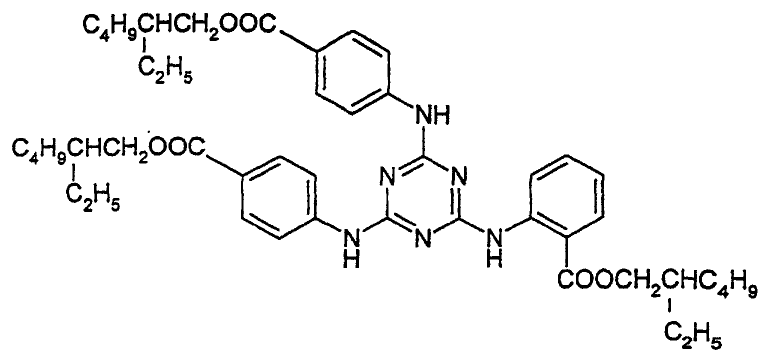

Examples 6-10 - Operating substantially as described in Example 5, reacting the suitable compound of formula IV with a compound of formula V, or a compound of formula VI in which X₁ is oxygen and the CO-X₁-R₁ group is at the para position, the following compounds of formula I were obtained

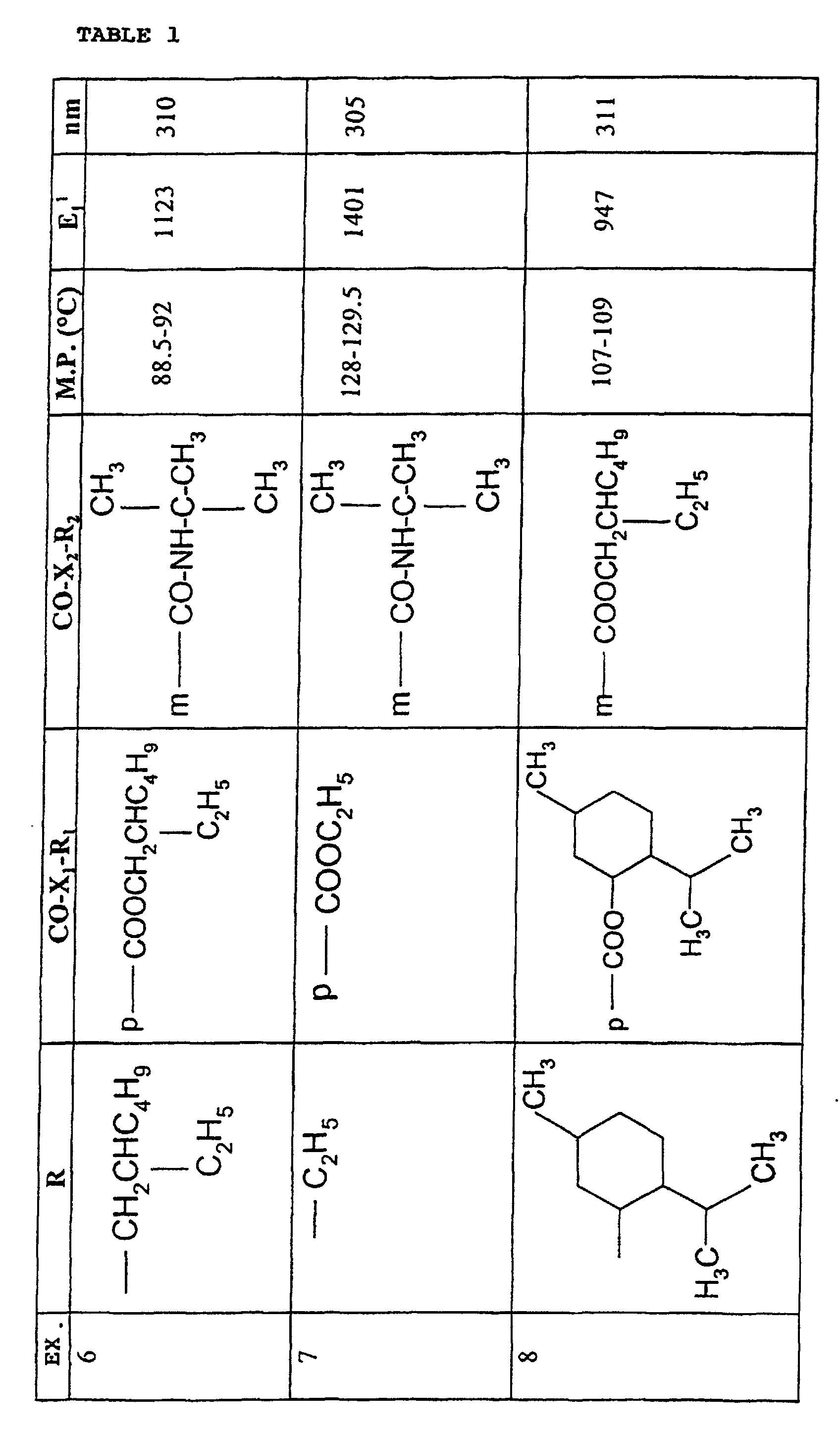

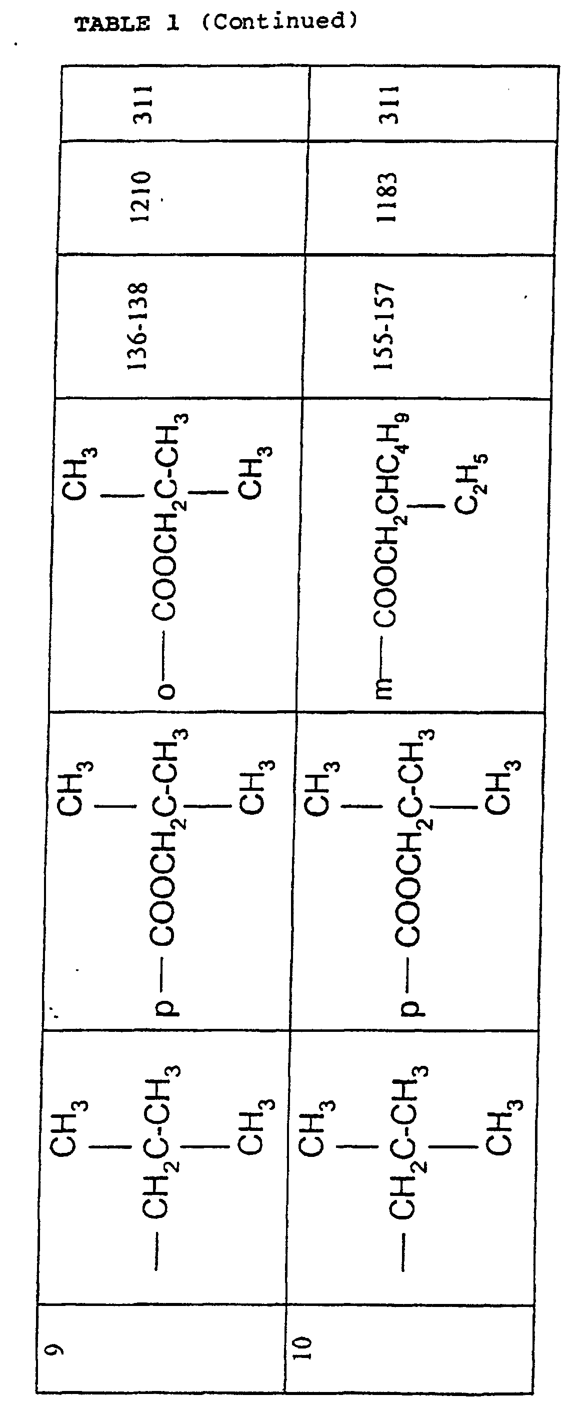

### Example A - Sun cream

| | |
|---|---|
| Polyglycol (Arlacel^{(R)} 165 ICI) | 2.0 g |
| Glycerin monostearate | 4.0 g |
| C₁₂₋₁₅ Alcohol benzoate | 5.0 g |
| Cetylstearyl alcohol | 3.0 g |
| Myristic alcohol with 3 mols of propylene oxide (Witcamol^{(R)} APM Witco) | 29.0 g |
| Compound of Example 7 | 2.0 g |
| 4-Methoxy-4'-tert.butyl-dibenzoylmethane | 2.5 g |
| Perfume | 0.3 g |
| Distilled water | 100.0 g |

The fatty phase is warmed to 80-90°C, the sunscreen of Example 7 is added, then the mixture is added to the water, containing the hydrosoluble compounds and the whole is heated to 80-90°C. Warm stirring is continued for 15-20 minutes. The mixture is slowly cooled and perfume is added.

### Example B - Sun milk

| | |
|---|---|
| Fatty acid triglycerides | 20.0 g |
| Cetylstearyl alcohol | 2.0 g |
| Lanolin | 4.0 g |
| Cetyl alcohol | 2.0 g |
| Silicone oil | 0.4 g |
| Compound of example 9 | 3.5 g |
| Abiol (preservative, 3V SIGMA Bergamo, Italy) | 0.2 g |
| Synthalen^{(R)} (thickening agent, 3V SIGMA Bergamo, Italy) | 0.1 g |
| Triethanolamine | 0.15 g |
| Perfume | 0.3 g |
| Distilled water | 100.0 g |

The sun milk is prepared substantially as described in example A.

### Example C - Lipsticks

First a base mixture is prepared consisting of:

| | |
|---|---|
| Beeswax | 13.0 g |
| Carnauba wax | 7.5 g |
| Lanolin | 5.0 g |
| Isopropyl myristate | 8.0 g |
| Mineral oil | 3.0 g |
| Castor oil | 63.5 g |

85 Grams of this mixture are melted, the melted mass is added with 5 g of compound of Example 7 and 8 g of 4-methoxy-4'-tert.butyl-dibenzoylmethane, as well as perfume, flavours and dyes; the mixture is then diluted to 1000 g with castor oil and cooled at room temperature.

**Example D** - 1000 Grams of low density polyethylene (Riblene^{(R)} EF 2100 R Enichem), 2 g of n-octadecyl-3-(3,5-di-tertbutyl-4-hydroxyphenyl)propionate, 1 g of calcium stearate and 0.3 grams of the compound of Example 10 are mixed homogeneously. The resulting mixture is extruded at 190°C and transformed into granules which are pressed at 200°C, to obtain 0.2 mm films.

Samples of these films are subjected to UV radiations in a Weatherometer WOM Ci-65 at a black panel temperature of 63°C. In the irradiated samples, the increase in the carbonyl band at 5.85 nm in the infrared is measured, and the time T 0.1 necessary to obtain a 0.1 increase in the carbonyl band is determined, compared with a film containing no compound of Example 10. Results are reported in table 2.

**Table 2**

| | |
|---|---|
| **Stabilizer** | **T 0.1** |
| No stabilizer | 340 |
| Compound of Example 10 | 1420 |

## Claims

1. Compounds of formula I: 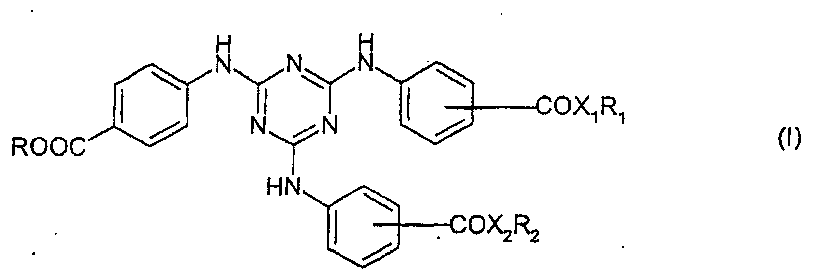 wherein R, R₁ and R₂ are independently straight or branched (C₁₋₁₈)alkyl; (C₅₋₁₂)cycloalkyl, optionally substituted with 1 to 3 straight or branched (C₁₋₄) alkyl groups;
X₁ and X₂ are independently oxygen or the group NH;
with the proviso that the two CO-X₁-R₁ and CO-X₂-R₂ groups cannot simultaneously be at the para position.

2. Compounds as defined in claim 1, in which R, R₁ and R₂ are independently straight or branched (C₁₋₁₈)alkyl, or (C₅-₁₂)cycloalkyl, optionally substituted with 1 to 2 straight or branched (C₁₋₄) alkyl groups, X₁ and X₂ are oxygen or NH, one of the two CO-X₁-R₁ or CO-X₂-R₂ groups is at the para position and the other is at a position different from the para position.

3. Compounds as defined in claim 1, in which R and R₁ are simultaneously straight or branched (C₁₋₁₈)alkyl, or (C₅₋₁₂)cycloalkyl, optionally substituted with 1 to 2 straight or branched (C₁₋₄) alkyl groups, R₂ is straight or branched (C₁₋₁₈)alkyl, or (C₅₋₁₂)cycloalkyl, optionally substituted with 1 to 2 straight or branched (C₁₋₄) alkyl groups, X₁ is oxygen, X₂ can be oxygen or NH, the CO-X₁-R₁ group is at the para position and the CO-X₂-R₂ group is at a position different from the para position.

4. The use of the compounds as claimed in claims 1-3 as sunscreens for the preparation of cosmetic compositions for protecting the skin from sunlight.

5. The use of the compounds as claimed in claims 1-4 as sunscreens for the cosmetic treatment of the skin.

6. A method for the protection of cosmetic compositions from the degradation induced by UV radiation, which comprises adding an effective amount of one or more compounds as claimed in claims 1-4, optionally in combination with other known stabilizers.

7. Cosmetic compositions containing one or more compounds as claimed in claims 1-4 in amounts ranging from about 0.1 to about 20% on the total weight of the composition.

8. Cosmetic compositions containing one or more compounds as defined in claims 1-4 in amounts ranging from about 0.5 to about 15% on the total composition weight.

9. Cosmetic compositions as defined in claims 7 and 8 comprising, in addition to the compounds as claimed in claims 1-4, other sunscreens against sun radiations, and/or inorganic sunscreens, and/or vitamins or derivatives thereof.

10. Cosmetic compositions as defined in claim 9, comprising, in addition to the compounds as claimed in claims 1-4, sunscreens selected from 3-(4-methylbenzylidene)-camphor; 2-ethylhexyl-(4-dimethylamino)-benzoate; 2-ethylhexyl-4-methoxycinnamate; isoamyl-4-methoxycinnamate; menthyl salicylate; octyl salicylate; 2-ethylhexyl-2-cyano-3,3-diphenylacrylate; 2,4,6-tris-[(4-carbo-2-ethylhexyloxy)anilino)]-1,3,5-triazine; 2,4-bis-[(4-carbo-2-ethylhexyloxy)anilino]-6-[(p.-tert.butylaminocarbonyl)amino]-1,3,5-triazine; 4-methoxy-4'-tert.butyldibenzoylmethane; 2-hydroxy-4-methoxybenzophenone; 2-phenylbenzimidazol-5-sulfonic acid and the salts thereof; 2-hydroxy-4-methoxy-benzophenone-5-sulfonic acid and the salts thereof; terephthalydene-3,3'-dicamphor-10,10'-disulfonic acid and the salts thereof; and/or titanium dioxide, zinc oxide, silica or aluminium oxide, and/or vitamins of the groups A, B, C, D, vitamin E, tocopherols, and derivatives thereof.

11. The use of the compounds as claimed in claims 1 to 4 as photostabilizers for synthetic polymers.

12. A method for the stabilization of synthetic polymers from UV radiation, which comprises adding an effective amount of at least one compound as claimed in claims 1-4, optionally in combination with other known stabilizers and additives for polymers.

13. Compositions of synthetic polymers containing from about 0.01 to about 5% by weight of one or more compounds as claimed in claims 1-4 on the weight of the polymer material.

14. Compositions of synthetic polymers containing from about 0.05 to about 2% by weight of one or more compounds as claimed in claims 1-4 on the weight of the polymer material.

15. Compositions of synthetic polymers as claimed in claims 13 and 14 comprising, in addition to one or more compounds as claimed in claims 1-4, other known stabilizers and additives for polymers.

## Patentansprüche

1. Verbindungen der Formel I 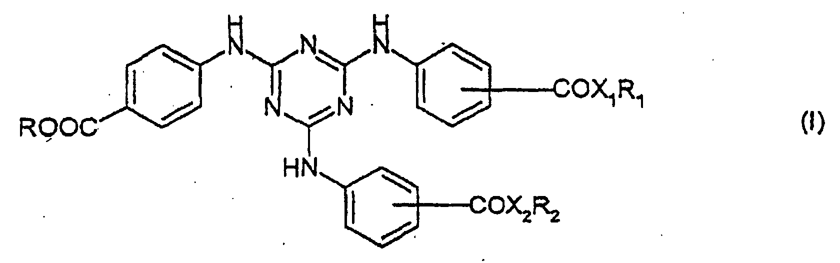 worin R, R₁ und R₂ unabhängig gerades oder verzweigtes (C₁₋₁₈)Alkyl; (C₅₋₁₂)Cycloalkyl, gegebenenfalls substituiert mit 1 bis 3 geraden oder verzweigten (C₁₋₄)Alkylgruppen sind;
X₁ und X₂ unabhängig Sauerstoff oder die Gruppe NH sind;
mit der Maßgabe, dass die beiden Gruppen CO-X₁-R₁ und CO-X₂-R₂ nicht gleichzeitig in der para-Stellung vorliegen können.

2. Verbindungen nach Anspruch 1, worin R, R₁ und R₂ unabhängig gerades oder verzweigtes (C₁₋₁₈)Alkyl oder (C₅₋₁₂)Cycloalkyl, gegebenenfalls substituiert mit 1 bis 2 geraden oder verzweigten (C₁₋₄)Alkylgruppen sind, X₁ und X₂ Sauerstoff oder NH sind, eine der beiden Gruppen CO-X₁-R₁ oder CO-X₂-R₂ in der para-Stellung und die andere in einer von der para-Stellung unterschiedlichen Stellung vorliegen.

3. Verbindungen nach Anspruch 1, worin R und R₁ gleichzeitig gerades oder verzweigtes (C₁₋₁₈)Alkyl oder (C₅₋₁₂)Cycloalkyl, gegebenenfalls substituiert mit 1 bis 2 geraden oder verzweigten (C₁-₄)Alkylgruppen sind, R₂ gerades oder verzweigtes (C₁₋₁₈)Alkyl oder (C₅-₁₂)Cycloalkyl, gegebenenfalls substituiert mit 1 bis 2 geraden oder verzweigten (C₁₋₄)Alkylgruppen ist, X₁ Sauerstoff ist, X₂ Sauerstoff oder NH sein kann, wobei die Gruppe CO-X₁-R₁ in der para-Stellung und die Gruppe CO-X₂-R₂ In einer von der para-Stellung unterschiedlichen Stellung vorliegen.

4. Verwendung der Verbindungen nach Anspruch 1 - 3 als Sonnenfilter für die Herstellung kosmetischer Zusammensetzungen zum Schutz der Haut vor Sonnenlicht.

5. Verwendung der Verbindungen gemäß Anspruch 1 - 4 als Sonnenfilter bzw. Sonnencreme für die kosmetische Behandlung der Haut.

6. Verfahren zum Schutz von kosmetischen Zusammensetzungen vor der durch UV-Strahlung bedingten Zersetzung, bei dem eine wirksame Menge einer oder mehrerer Verbindungen gemäß den Ansprüchen 1 - 4, gegebenenfalls in Kombination mit anderen bekannten Stabilisatoren zugesetzt werden.

7. Kosmetische Zusammensetzungen, enthaltend eine oder mehrere Verbindungen gemäß Anspruch 1 - 4 in Mengen im Bereich von etwa 0,1 bis etwa 20 % des Gesamtgewichts der Zusammensetzung.

8. Kosmetische Zusammensetzungen, enthaltend eine oder mehrere Verbindungen gemäß Anspruch 1 - 4 in Mengen im Bereich von etwa 0,5 bis etwa 15 % bezogen auf das Gesamtgewicht der Zusammensetzung.

9. Kosmetische Zusammensetzungen gemäß Anspruch 7 und 8, die zusätzlich zu den Verbindungen gemäß Anspruch 1 - 4 andere Sonnenfilter gegen Sonnenstrahlung und/oder anorganische Sonnenfilter und/oder Vitamine oder deren Derivate enthalten.

10. Kosmetische Zusammensetzungen gemäß Anspruch 9, enthaltend zusätzlich zu den Verbindungen gemäß Anspruch 1 - 4 Sonnenfilter, ausgewählt aus 3-(4-Methylbenzyliden)-Kampher; 2-Ethylhexyl-(4-dimethylamino)-benzoat; 2-Ethylhexyl-4-methoxycinnamat; Isoamyl-4-methoxycinnamat; Methylsalicylat; Octylsalicylat; 2-Ethylhexyl-2-cyano-3,3-diphenylacrylat; 2,4,6-Tris-[(4-carbo-2-ethylhexyloxy)anilino)]-1,3,5-triazin; 2,4-Bis-[(4-carbo-2-ethylhexyloxy)anilino]-6-[(p.-tert.butylaminocarbonyl)amino]-1,3,5-triazin; 4-Methoxy-4'-tert.butyl-dibenzoylmethan; 2-Hydroxy-4-methoxybenzophenon; 2-Phenyl-benzimidazol-5-sulfonsäure und deren Salze; 2-Hydroxy-4-methoxy-benzophenon-5-sulfonsäure und deren Salze; Terephthalyden-3,3'-dikampher-10,10'-disulfonsäure und deren Salze; und/oder Titandioxid, Zinkoxid, Siliziumdioxid oder Aluminiumoxid und/oder Vitamine der Gruppen A, B, C, D, Vitamin E, Tocopherole und Derivate davon.

11. Verwendung der Verbindungen gemäß Anspruch 1 bis 4 als Photostabilisatoren für synthetische Polymere.

12. Verfahren zur Stabilisierung von synthetischen Polymeren gegen UV-Strahlung durch Zusatz einer wirksamen Menge mindestens einer Verbindung gemäß Ansprüchen 1 - 4, gegebenenfalls in Kombination mit anderen bekannten Stabilisatoren und Additiven für Polymere.

13. Zusammensetzungen von synthetischen Polymeren, enthaltend etwa 0,01 bis etwa 5 Gew.-% einer oder mehrerer Verbindungen gemäß Ansprüchen 1 - 4 bezogen auf das Gewicht des Polymermaterials.

14. Zusammensetzungen von synthetischen Polymeren, enthaltend etwa 0,05 bis etwa 2 Gew.-% einer oder mehrerer Verbindungen gemäß Ansprüchen 1 - 4 bezogen auf das Gewicht des Polymermaterials.

15. Zusammensetzungen von synthetischen Polymeren gemäß Anspruch 13 und 14 enthaltend zusätzlich zu einer oder mehreren Verbindungen gemäß Ansprüchen 1 - 4 andere bekannte Stabilisatoren und Additive für Polymere.

## Revendications

1. Composés de formule I : 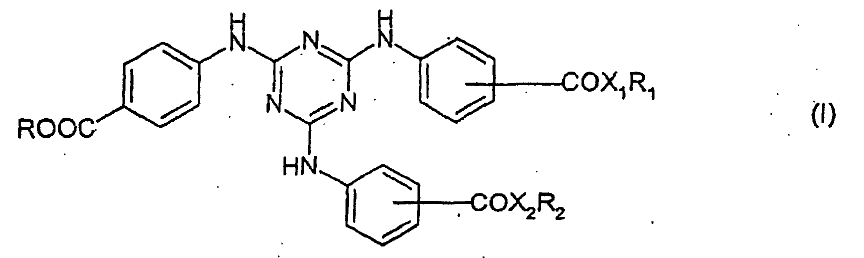 dans laquelle
R, R₁ et R₂ sont chacun indépendamment un groupe alkyle linéaire ou ramifié en C₁-C₁₈ ; un groupe cycloalkyle en C₅-C₁₂ facultativement substitué par 1 à 3 groupes alkyle linéaires ou ramifiés en C₁-C₄ ;
X₁ et X₂ sont chacun indépendamment de l'oxygène ou le groupe NH ;
avec la condition que les deux groupes CO-X₁-R₁ et CO-X₂-R₂ ne soient pas simultanément à la position para.

2. Composés tels que définis dans la revendication 1, dans lesquels R, R₁ et R₂ sont chacun indépendamment un groupe alkyle linéaire ou ramifié en C₁-C₁₈ ou un groupe cycloalkyle en C₅-C₁₂ facultativement substitué par 1 à 2 groupes alkyle linéaires ou ramifiés en C₁-C₄, X₁ et X₂ sont de l'oxygène ou NH, l'un des deux groupes CO-X₁-R₁ ou CO-X₂-R₂ est à la position para et l'autre est à une position différente de la position para.

3. Composés tels que définis dans la revendication 1, dans lesquels R et R₁ sont chacun simultanément un groupe alkyle linéaire ou ramifié en C₁-C₁₈ ou un groupe cycloalkyle en C₅-C₁₂ facultativement substitué par 1 à 2 groupes alkyle linéaires ou ramifiés en C₁-C₄, R₂ est un groupe alkyle linéaire ou ramifié en C₁-C₁₈ ou un groupe cycloalkyle en C₅-C₁₂ facultativement substitué par 1 à 2 groupes alkyle linéaires ou ramifiés en C₁-C₄, X₁ est de l'oxygène, X₂ peut être de l'oxygène ou NH, le groupe CO-X₁-R₁ est à la position para et le groupe CO-X₂-R₂ est à une position différente de la position para.

4. L'utilisation des composés tels que revendiqués dans les revendications 1 à 3 comme écrans solaires pour la préparation de compositions cosmétiques destinées à protéger la peau contre la lumière solaire.

5. L'utilisation des composés tels que revendiqués dans les revendications 1 à 4 comme écrans solaires pour le traitement cosmétique de la peau.

6. Un procédé pour la protection de compositions cosmétiques contre la dégradation provoquée par un rayonnement UV, qui comprend l'addition d'une quantité efficace d'un ou plusieurs composés tels que revendiqués dans les revendications 1 à 4, facultativement en association avec d'autres stabilisants connus.

7. Compositions cosmétiques contenant un ou plusieurs composés tels que revendiqués dans les revendications 1 à 4 en des quantités comprises entre environ 0,1 et environ 20 % du poids total de la composition.

8. Compositions cosmétiques contenant un ou plusieurs composés tels que définis dans les revendications 1 à 4 en des quantités comprises entre environ 0,5 et environ 15 % du poids total de la composition.

9. Compositions cosmétiques telles que définies dans les revendications 7 et 8 comprenant, en plus des composés tels que revendiqués dans les revendications 1 à 4, d'autres écrans solaires contre les rayons solaires, et/ou des écrans solaires minéraux, et/ou des vitamines ou leurs dérivés.

10. Compositions cosmétiques telles que définies dans la revendication 9, comprenant, en plus des composés tels que revendiqués dans les revendications 1 à 4, des écrans solaires choisis parmi le 3-(4-méthylbenzylidène)-camphre ; le (4-diméthylamino)benzoate de 2-éthylhexyle ; le 4-méthoxycinnamate de 2-éthylhexyle ; le 4-méthoxycinnamate d'isoamyle ; le salicylate de menthyle ; le salicylate d'octyle ; le 2-cyano-3,3-diphénylacrylate de 2-éthylhexyle ; la 2,4,6-tris[(4-carbo-2-éthylhexyloxy)anilino)]-1,3,5-triazine ; la 2,4-bis[(4-carbo-2-éthylhexyloxy)-anilino]-6-[(p.-*tert*-butylaminocarbonyl)amino]-1,3,5-triazine ; le 4-méthoxy-4'-*tert*-butyldibenzoylméthane ; la 2-hydroxy-4-méthoxybenzophénone ; l'acide 2-phénylbenzimidazole-5-sulfonique et ses sels ; l'acide 2-hydroxy-4-méthoxy-benzophénone-5-sulfonique et ses sels ; l'acide téréphtalylidène-3,3'-dicamphre-10,10'-disulfonique et ses sels ; et/ou le bioxyde de titane, l'oxyde de zinc, la silice ou l'oxyde d'aluminium, et/ou des vitamines des groupes A, B, C, D, la vitamine E, des tocophérols, et leurs dérivés.

11. L'utilisation des composés tels que revendiqués dans les revendications 1 à 4 comme photostabilisants pour polymères synthétiques.

12. Un procédé pour la stabilisation de polymères synthétiques vis-à-vis du rayonnement UV, qui comprend l'addition d'une quantité efficace d'au moins un composé tel que revendiqué dans les revendications 1 à 4, facultativement en association avec d'autres stabilisants et additifs connus pour polymères.

13. Compositions de polymères synthétiques contenant environ 0,01 à environ 5 % en poids d'un ou plusieurs composés tels que revendiqués dans les revendications 1 à 4 par rapport au poids de la matière polymère.

14. Composition de polymères synthétiques contenant environ 0,05 à environ 2 % en poids d'un ou plusieurs composés tels que revendiqués dans les revendications 1 à 4 par rapport au poids de la matière polymère.

15. Compositions de polymères synthétiques telles que revendiquées dans les revendications 13 et 14 comprenant, en plus d'un ou plusieurs composés tels que revendiqués dans les revendications 1 à 4, d'autres stabilisants et additifs connus pour polymères.
